(19) Europäisches Patentamt
European Patent Office
Office européen des brevets

(11) Publication number : **0 451 941 B1**

# (12) EUROPEAN PATENT SPECIFICATION

(45) Date of publication of patent specification : **30.06.93 Bulletin 93/26**

(51) Int. Cl.$^5$ : **B01J 29/28,** C07C 2/00

(21) Application number : **91301666.3**

(22) Date of filing : **28.02.91**

## (54) Catalyst and process for the selective production of para-dialkyl substituted benzenes.

(30) Priority : **05.06.90 US 533150**
**21.03.90 US 497046**

(43) Date of publication of application :
**16.10.91 Bulletin 91/42**

(45) Publication of the grant of the patent :
**30.06.93 Bulletin 93/26**

(84) Designated Contracting States :
**BE DE FR GB IT NL**

(56) References cited :
**EP-A- 0 030 811**
**EP-A- 0 289 691**
**US-A- 4 175 114**
**US-A- 4 465 886**

(73) Proprietor : **MOBIL OIL CORPORATION**
**3225 Gallows Road**
**Fairfax, Virginia 22037-0001 (US)**

(72) Inventor : **Absil, Robert Peter Leonard**
**811 St. Regis Court**
**W. Deptford, NJ 08051 (US)**
Inventor : **Chang, Clarence Dayton**
**11 Murray Place**
**Princeton, NJ 08540 (US)**
Inventor : **Han, Scott**
**4 Valerie Lane**
**Lawrenceville, NJ 08648 (US)**
Inventor : **Marler, David Owen**
**801 Cooper Street, No. 272B**
**Deptford, NJ 08096 (US)**
Inventor : **Mitko, Donna**
**1607 Georges Road**
**Monmouth Junction, NJ 08852 (US)**
Inventor : **Shihabi, David Said**
**Harbourton-Woodsville Road, RR1, Box 334A**
**Pennington, NJ 08534 (US)**

(74) Representative : **Colmer, Stephen Gary et al**
**Patent Department c/o Mobil Services**
**Company Limited Mobil Court 3 Clements Inn**
**London WC2A 2EB (GB)**

EP 0 451 941 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed to a catalyst and a process for the selective production of para-dialkyl substituted benzenes, and in particular, for the selective disproportionation of toluene to para-xylene.

Processes for the selective production of para-dialkyl substituted benzenes are known in the art. For example, U.S. Patent No. 4,090,981 to Rodewald discloses a method for making a catalyst particularly suitable for the selective production of para-dialkyl substituted benzenes wherein a porous crystalline aluminosilicate zeolite is coated with a silica and then subjected to heating in an oxygen containing atmosphere at temperatures in excess of 300°C. U.S. Patent No. 4,117,026 to Haag et al. discloses the production of dialkylbenzenes by the disproportionation of monoalkylbenzenes over zeolites pretreated with difficultly reducible oxides, including magnesium oxide, or coke. U.S. Patent No. 4,465,886 to Rodewald also discloses a silica-modified catalyst for use in the selective production of para-dialkyl substituted benzenes. U.S. Patent No. 4,477,583 to Rodewald relates to a method of preparing a composition having a crystalline zeolite with a coating of silica which is useful in the selective production of para-dialkyl substituted benzenes. These patents contemplate the use of zeolite catalysts synthesized in the presence of organonitrogen templates. Such catalysts, hereinafter referred to as "organic catalysts", are disclosed in U.S. Patent No. 3,702,886 to Argauer et al.

In one aspect, the present invention is directed to a catalyst for the selective production of para-dialkyl substituted benzenes, comprising a zeolite which:

a) has a Constraint Index of 1-12;

b) has been produced from a forming mixture free of organic directing agent; and

c) has been modified by treatment with an organosilicon compound.

In a further aspect, the present invention resides in the use of the catalyst of said one aspect of the invention in the selective production of para-dialkyl substituted benzenes.

The process of the invention provides greater para-selectivity for a given conversion than the processes of the prior art. The process of the present invention is also useful in obtaining para-dialkyl substituted benzenes in high selectivity at lower temperatures than previously available using processes known in the art.

The catalyst of the present invention employs a zeolite which is inherently shape-selective in that it exhibits a Constraint Index of 1-12. Constraint Index and the method by which it is determined are described U.S. Patent 4,016,218. The Constraint Index (CI) values for some typical zeolites are:

| | CI | (at test temperature) |
|---|---|---|
| ZSM-4 | 0.5 | (316°C) |
| ZSM-5 | 6-8.3 | (371°C-316°C) |
| ZSM-11 | 5-8.7 | (371°C-316°C) |
| ZSM-12 | 2.3 | (316°C) |
| ZSM-20 | 0.5 | (371°C) |
| ZSM-22 | 7.3 | (427°C) |
| ZSM-23 | 9.1 | (427°C) |
| ZSM-34 | 50 | (371°C) |
| ZSM-35 | 4.5 | (454°C) |
| ZSM-48 | 3.5 | (538°C) |
| ZSM-50 | 2.1 | (427°C) |
| TMA Offretite | 3.7 | (316°C) |
| TEA Mordenite | 0.4 | (316°C) |
| Clinoptilolite | 3.4 | (510°C) |
| Mordenite | 0.5 | (316°C) |
| REY | 0.4 | (316°C) |
| Amorphous Silica-alumina | 0.6 | (538°C) |
| Dealuminized Y | 0.5 | (510°C) |
| Erionite | 38 | (316°C) |
| Zeolite Beta | 0.6-2.0 | (316°C-399°C) |

Zeolites useful in the present invention have a Constraint Index within the reange 1-12 at some temperature within the 290°C to 538°C.

Suitable zeolites for use in the present invention include ZSM-5, ZSM-11, ZSM-12, ZSM-22, ZSM-23, ZSM-35, ZSM-48, ZSM-50 and Beta, of which ZSM-5 is especially preferred. ZSM-5 is described in U.S. Patent 3,702,886. ZSM-11 is described in U.S. Patent 3,709,979. ZSM-12 is described in U.S. Patent 3,832,449. ZSM-22 is described in Canadian Patent No. 1,210,747. ZSM-23 is described in U.S. Patent 4,076,842. ZSM-35 is described in U.S. Patent 4,016,245. ZSM-48 is described in U.S. Patent 4,397,827. ZSM-50 is described in U.S. Patent 4,640,849. Zeolite beta is described in U.S. Patent 3,308,069.

To achieve the selectivity and activity required in the present invention, the zeolite used is synthesized from a forming mixture free of organic directing agent. Thus, for example, although ZSM-5 is frequently prepared in the presence of tetrapropylammonium cations, the synthesis of ZSM-5 in the absence of organic directing agents is also well-known and is, for example, described in U.S. Patent Nos. 4,175,114 and 4,257,885. Preferably, where the zeolite used is ZSM-5, the synthesis mixture is not only free of organic directing agent, but also has a solids content in excess of 20% wt, and preferably 25 to 40% wt, of the synthesis mixture and employs a silica souce produced by continuous precipitation, preferably with acid, from an aqueous silicate solution. The preparation of such a precipitated silica source and its use in the synthesis of ZSM-5 is described in EP-A-202797.

Preferably the zeolite employed in the present invention has a silica to alumina mole ratio less than 55:1 and more preferably less than 30:1.

The zeolite employed in preparing the catalyst of the invention preferably exhibits a diffusion rate constant ($D/r^2$) of less than about 150 $sec^{-1} \times 10^{-6}$, preferably less than 120 $sec^{-1} \times 10^{-6}$ and most preferably less than 100 $sec^{-1} \times 10^{-6}$. Diffusivities and diffusion rates herein are determined by measuring the time ($t_{0.3}$) it takes to sorb 30% of o-xylene (of total o-xylene capacity) by the determination described in U.S. Patent 4,117,026. The characteristic diffusion time, $t_{0.3}$, is a direct measure of the critical mass transfer property $r^2/D$, where D is the diffusion co-efficient ($cm^2/sec$) and r= the crystal radius (cm) and is determinable by experiment.

Diffusion parameters are calculated from sorption rates by assuming that the plane sheet model describes the diffusion process. For every sorbate loading $Q/Q_\infty = 0.3$ (for 30% o-xylene of total o-xylene capacity), where $Q_\infty$, is the equilibrium sorbate loading, there is a $(Dt/r^2)^{1/2}$ value where D, t, and r are the diffusion coefficient ($cm^2/sec$) , time (sec) [which is determinable by experiment], and crystal radius (cm), respectively. Hence from the sorption time required to reach a given sorbate loading, $D/r^2$ can be calculated directly.

Sorption measurements are carried out with a Dupont Instruments Thermogravimetric Analyzer Model 951 and Sage Instruments Syring Pump Model 355. Xylene sorption and diffusion measurements are made by heating a fresh 50 mg sample of hydrogen form zeolite in flowing nitrogen at 500°C to a constant weight. The temperature is reduced to 120°C, and either ortho- or para-xylene is delivered to the TGA module by means of the syring pump. The hydrocarbon is instantaneously vaporized and swept over the zeolite by a 60 cc/min nitrogen purge. Para-xylene is used to obtain equilibrium xylene capacity due to the slow equilibrium approach of ortho-xylene.

Prior to use in the present invention the zeolite is modified by treatment with an organosilicon compound, which preferably has a molecular size such that it is substantially incapable of entering the pores of the zeolite. Suitable organosilicon compounds obey the general formula:

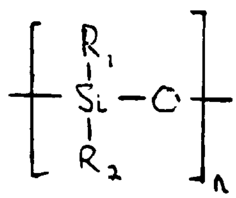

where $R_1$ is hydrogen, flourine, hydroxy, alkyl, aralkyl, alkaryl or fluoro-alkyl.
The hydrocarbon substituents generally contain from 1 to 10 carbon atoms and preferably are methyl or ethyl groups. $R_2$ is selected from the same group as $R_1$, other than hydrogen and other than methyl if $R_1$ is hydrogen and n is an integer of at least 10 and generally in the range of 10 to 1000. The molecular weight of the silicone compound employed is generally between 500 and 20,000 and preferably between 1000 and 10,000. Representative silicone compounds include dimethylsilicone, diethylsilicone, phenylmethylsilicone, ethylhydrogensilicone, phenylhydrogensilicone, methylethylsilicone, phenylethylsilicone, diphenylsilicone, methyltrifluoropropylsilicone, ethyltrifluoropropylsilicone, polydimethylsilicone, tetrachlorophenylmethyl silicone, tetrachlorophenylethyl silicone, tetrachlorophenylhydrogen silicone, tetrachlorophenylphenyl silicone, methylvinylsilicone and ethylvinylsilicone.

Alternatively, the organoosilicon compound may be a siloxane, an alkyl and/or alkoxysilane, or a polysilane.

Modification of the zeolite can be accomplished by contacting the zeolite with a solution of the organosilicon compound in an organic solvent, typically at 10-200°C for 0.2-5 hours, and then evaporating off the solvent. Alternatively, the zeolite can be contacted with an aqueous emulsion of the organosilicon compound, and then the water can be evaporated. In either case, the silicone treated zeolite is then calcined in air, preferably at 350-550°C for 1-24 hours, to yield a silica-coated zeolite containing 0.5-30 wt%, preferably 1-15 wt% silica.

The catalyst of the present invention is stable and regenerable. Thus the "selectivation" achieved by deposition of the silica coating is permanent and substantially unaffected by catalyst regeneration. In contrast, coke-selectivated catalysts, such as those disclosed in U.S. Patent No. 4,117,026, require pre-coking after each regeneration cycle.

Prior to use in the process of the invention, the silica-coated zeolite is preferably combined with a support or binder material such as, for example, a porous inorganic oxide support or a clay binder. Examples of suitable binder materials include alumina, germania, zirconia, silica, magnesia, thoria, titania, boria and combinations thereof, generally in the form of dried inorganic oxide gels and gelatinous precipitates. In general, however, it is preferred to employ low acidity refractory oxide binders, such as silica, germania, titania, or zirconia, preferably silca. The relative proportion of zeolite in the total composition of catalyst and binder or support may vary widely with the zeolite content ranging from 30 to 90% by weight and more usually from 50 to 80% by weight of the composition. The composition may be in the form of an extrudate, beads or fluidizable microspheres.

The catalyst of the invention is suitable for the alkylation of benzene or substituted benzene compounds, as well as the disproportionation of substituted benzene compounds. Preferably, the substituted benzene compound is an alkyl-substituted benzene compound having 1-4 carbon atoms in the alkyl substituent and the alkylating agent contains from 1 to 4 carbon atoms. The catalyst is particularly suitable for the selective disproportionation of toluene to p-xylene.

The process of the present invention may be practiced over a range of reaction conditions such as a temperature of 260 to 650°C (500 to 1200°F), preferably 315 to 480°C (600 to 900°F), a pressure of 1480 to 7000 kPa (200 to 1000 psig), preferably 2860 to 5620 kPa (400 to 800 psig), and a weight hourly space velocity (WHSV) of 1/2 to 20, preferably 4 to 10. The weight hourly space velocity is the weight of liquid flowing through the reactor every hour divided by the weight of zeolite in the catalyst.

The invention will now be more particularly described with reference to the following examples and the accompanying drawing which provides graphs comparing the selectivity and activity of fresh and regenerated catalyst of the present invention.

## EXAMPLE 1 (COMPARATIVE)

An organic ZSM-5 zeolite was prepared according to U.S. Patent No. 3,702,886, using a tetrapropylammonium bromide directing agent. The ZSM-5 product analysis showed Si02/A1203 = 25.4 molar ratio. This zeolite was silica-extruded to form an extrudate having 65 wt% ZSM-5 and 35 wt% silica. The extrudate was calcined in nitrogen at 540°C (1000°F) for 5-6 hrs. and then ion exchanged with 1N NH3N03 at room temperature overnight, dried at 130°C (266°F), and calcined in air, raising the furnace temperature 1°C/min. to 540°C (1000°F), and holding at this temperature 5-6 hrs., thus converting the zeolite component into the hydrogen form. To 0.6 grams of phenylmethylsilicone (molecular weight ca. 1686) dissolved in 20 cc of hexane was added 2.5 grams of this extrudate. The mixture was heated to 20°C (68°F) to remove hexane solvent, and the residue calcined in air by raising the temperature 1°C/min to 540°C (1000°F) and holding at this temperature for 7 hours. The product was a silica-coated catalyst containing 10 wt% modifier silica.

## EXAMPLE 2

A non-organic ZSM-5 zeolite was prepared according to U.S. Patent No. 4,175,114. The ZSM-5 product analysis showed Si02/Al203 = 26 mole ratio. This zeolite was silica-extruded, converted to the hydrogen form, and treated with silicone in a manner identical to Example 1.

## EXAMPLE 3

A catalyst was prepared according to Example 2 with the exception that extrusion was effected using alumina, in place of silica, as the binder. The finished catalyst contained 10 wt% modifier silica.

## EXAMPLE 4

A catalyst was prepared according to Example 3 with the exception that double the amount of phenylmethylsilicone was utilized, yielding a finished catalyst containing 20 wt% modifier silica.

## EXAMPLE 5

A catalyst was prepared according to Example 2 with the exception that dimethylsilicone (molecular weight 4835, 0.4 g. in 20 cc. hexane per 2.5 g. extrudate) was used in place of phenylmethylsilicone, yielding a finished catalyst containing 10 wt% modifier silica.

Toluene disproportionation activity of each of the catalysts was tested in a micro-unit. In each run, 2 to 2.3 grams of the 1/16" extrudate catalyst were mixed with 4-5 grams of sand. The mixtures were then charged to stainless steel reactors having outside diameters of 1cm and the runs performed under conditions of a pressure of 3550 kPa (500 psig), 4-8 WHSV (zeolite), 2H2/HC, and a temperature of 363-482°C (685-900°F). The toluene used was purified by percolating through activated alumina. Liquid and gas products were analyzed by gas chromatography.

Table 1 contains results of toluene disproportionation using the silicone- modified organic zeolite catalyst of Example 1. It is evident that over a range of reaction conditions the p-xylene selectivity is close to equilibrium. Table 2 contains results of selective toluene disproportionation over the silica-bound, silicone-modified non-organic zeolite catalysts of Example 2 and Example 5. Included for comparison are results obtained using an unmodified silica-bound non-organic catalyst. It is clear that the silicone-modified catalysts gave vastly superior results, affording xylene para-selectivities far in excess of equilibrium. Table 3 contains results using the alumina-bound, silicone-modified non-organic catalysts of Examples 3 and 4. Included for comparison are results obtained using an unmodified alumina-bound, non-organic catalyst. These results demonstrate that compared to silica-bound catalyst, higher loadings of modifier silica are necessary to achieve high xylene para-selectivity.

TABLE 1

| Days on Stream | 03 | 05 | 06 | 11 | 12 | 13 |
|---|---|---|---|---|---|---|
| WHSV | 2.2 | 4.0 | 4.0 | 4.0 | 4.0 | 4.0 |
| $H_2$/HC (mole) | 2/1 | 2/1 | 2/1 | 2/1 | 2/1 | 2/1 |
| Temperature (F) | 739 | 831 | 878 | 880 | 900 | 900 |
| (C) | 393 | 444 | 470 | 471 | 482 | 482 |
| Conversion (wt%) | 7.4 | 19.0 | 31.7 | 26.1 | 31.3 | 30.9 |
| Liquid Yield (wt%) | 100 | 99.6 | 99.1 | 99.6 | 98.6 | 98.6 |
| Benzene/Xylenes | 1.04 | 1.08 | 1.11 | 1.06 | 1.11 | 1.11 |
| Benzene Selec (%) | 40.9 | 42.3 | 42.0 | 41.1 | 41.5 | 41.4 |
| Xylenes Selec (%) | 53.3 | 53.2 | 51.5 | 52.7 | 50.8 | 50.7 |
| $C_5^-$ | 0.00 | 0.06 | 0.26 | 0.11 | 0.53 | 0.54 |
| Benzene | 3.02 | 8.03 | 13.32 | 10.74 | 12.98 | 12.79 |
| Ethylbenzene | 0.00 | 0.06 | 0.22 | 0.17 | 0.26 | 0.26 |
| p-Xylene | 0.97 | 2.57 | 4.11 | 3.50 | 4.02 | 3.96 |
| m-Xylene | 2.02 | 5.10 | 8.20 | 7.00 | 8.07 | 7.06 |
| o-Xylene | 0.94 | 2.34 | 3.92 | 3.26 | 3.81 | 3.75 |
| $C_9^+$ | 0.43 | 0.74 | 1.58 | 1.32 | 1.64 | 1.62 |
| Total Xylenes | 3.93 | 10.10 | 16.32 | 13.76 | 15.90 | 15.67 |
| p-Xylene selectivity, % | 24.6 | 25.4 | 25.2 | 25.4 | 25.3 | 25.3 |
| Theoretical equilibrium | 23.6 | 23.4 | 23.3 | 23.3 | 23.2 | 23.2 |

TABLE 2

| Catalyst | Unmodified | | | Modified | | | | | | |
|---|---|---|---|---|---|---|---|---|---|---|
| | Silica-Bound ZSM-5 | | | Example 2 | | | | Example 5 | | |
| Modification | None | | | Phenylmethyl-silicone | | | | Dimethyl-silicone | | |
| Days on Stream | 1 | 2 | 92 | 1 | 3 | 17 | 63 | 2 | 3 | 9 |
| WHSV | 4 | 6 | 8 | 4 | 4 | 7 | 7 | 6 | 6 | 6 |
| Temperature °F | 739 | 739 | 760 | 739 | 739 | 781 | 792 | 808 | 826 | 822 |
| °C | 393 | 393 | 404 | 393 | 393 | 416 | 422 | 431 | 441 | 439 |
| Toluene Conv., wt% | 34.5 | 26.2 | 30.9 | 30.4 | 30 | 29.5 | 30 | 27.5 | 33.1 | 28.6 |
| Yields, wt% | | | | | | | | | | |
| $C_5^-$ | 1.0 | 0.5 | 0.3 | 1.7 | 1.3 | 0.9 | 1.1 | 0.4 | 1.4 | 0.7 |
| Xylenes | 17.5 | 13.7 | 17.0 | 14.9 | 14.6 | 14.5 | 15.0 | 14.6 | 16.7 | 14.8 |
| $C_9^+$ | 1.4 | 1.0 | 0.7 | 0.4 | 0.5 | 0.5 | 0.5 | 0.8 | 1.2 | 0.9 |
| p-Xylene | 24.9 | 25.1 | 26.4 | 65.6 | 67.9 | 75.5 | 86.0 | 58.0 | 54.1 | 56.9 |
| Theoretical Equilibrium | 23.6 | 23.6 | 23.5 | 23.6 | 23.6 | 23.5 | 23.4 | 23.4 | 23.4 | 23.4 |

TABLE 3

| Catalyst | Unmodified | Modified | | | |
|---|---|---|---|---|---|
| | Alumina-Bound ZSM-5 | Example 3 | | Example 4 | |
| Modification | None | Phenylmethyl-silicone (10% modifier (silica) | | Phenylmethyl-silicone (20% modifier silica) | |
| Days on Stream | 2 | 2 | 15 | 2 | 3 |
| WHSV | 4 | 4 | 7 | 4 | 4 |
| Temperature °F | 685 | 754 | 795 | 740 | 779 |
| °C | 363 | 401 | 424 | 393 | 415 |
| Toluene Conv., wt% | 28.0 | 30.3 | 30.2 | 21.0 | 32.0 |
| Yields, wt% | | | | | |
| $C_5^-$ | 0.4 | 0.6 | 0.5 | 0.3 | 0.3 |
| Xylenes | 14.9 | 15.6 | 16.1 | 11.2 | 17.2 |
| $C_9^+$ | 0.9 | 1.0 | 1.0 | 0.4 | 0.7 |
| p-Xylene selectivity | 24 | 27.6 | 29.1 | 54 | 49 |
| Theoretical equilibrium | 23.7 | 23.5 | 23.5 | 23.6 | 23.5 |

EXAMPLE 6

The activity, p-xylene selectivity, aging characteristics, and regenerability of the catalyst of Example 2 are shown in Figure 1 and Table 4. In this experiment, the first cycle was arbitrarily terminated after 63 days on stream, although the catalyst was still fully active, in order to demonstrate its regenerability: The catalyst was regenerated by calcination in air, with the temperature being increased 1°C/min to 540°C (1000°F) and held for 4 hrs. As seen in Figure 1 and Table 4, the catalytic performance of the regenerated catalyst substantially reproduces that of the fresh catalyst. It is further evident from Figure 1, that the catalyst of the instant invention has a dramatic activity advantage over the coke-selectivated catalysts of the prior art. Table 5 compares selectivities achieved using the instant catalyst with typical results of a coke-selectivated ZSM-5.

TABLE 4

SELECTIVE TOLUENE DISPROPORTIONATION OVER FRESH VS. REGENERATED SILICA-BOUND ORGANIC CATALYSTS

| Catalyst | Fresh | | Regenerated | |
|---|---|---|---|---|
| Days on Stream | 8 | 17.5 | 8 | 17 |
| WHSV | 7 | 7 | 7 | 7 |
| Temperature °F | 781 | 781 | 790 | 790 |
| °C | 416 | 416 | 421 | 421 |
| Toluene Conv,. wt% | 30.0 | 29.5 | 31.0 | 30.4 |
| Yields, wt% | | | | |
| $C_5$- | 1.3 | 0.9 | 1.3 | 1.5 |
| Xylenes | 14.3 | 14.5 | 14.7 | 15.4 |
| $C_9$+ | 0.4 | 0.5 | 0.8 | 0.8 |
| p-Xylene selectivity | 72.1 | 75.5 | 69.1 | 71.7 |

EXAMPLE 7

A non-organic ZSM-5 zeolite prepared according to U.S. Patent No. 4,175,114, is silica-extruded, and converted to the hydrogen form in a manner identical to Example 1. This is mixed with a 9% aqueous emulsion of phenylmethylsilicone to yield, after removal of water and calcination at 540°C (1000°F), a catalyst containing 9 wt% modifier silica. The catalyst is used for toluene disproportionation, giving 85% p-xylene selectivity at 420°C (790°F) and 30% conversion.

## Claims

1. A catalyst for the selective production of para-dialkyl substituted benzenes, comprising a zeolite which:
   a) has a Constraint Index of 1-12;
   b) has been produced from a forming mixture free of organic directing agent; and
   c) has been modified by treatment with an organosilicon compound.

2. A catalyst according to claim 1 wherein said zeolite comprises ZSM-5.

3. A catalyst according to claim 1 or claim 2 wherein the zeolite has a silica to alumina mole ratio less than 55:1.

4. A catalyst according to claim 1 or claim 2 wherein the zeolite has a silica to alumina mole ratio less than 30:1.

5. A catalyst according to any preceding claim wherein the zeolite-exhibits a diffusion rate constant ($D/r^2$) of less than 150 $sec^{-1}$ x $10^{-6}$.

6. A catalyst according to any preceding claim wherein modification of the zeolite includes heating the organosilicon-modified zeolite in an oxidising environment to convert the organosilicon compound to silica.

7. A catalyst according to claim 6 wherein the the mofified zeolite contains 0.5-30 wt % of silica.

8. A catalyst according to any preceding claim and including a binder selected from silica, germania, titania and zirconia.

9. A process for the selective production of a para dialkyl-substituted benzene by disproportionation of a mono alkyl-substituted benzene having 1-4 carbon atoms in the alkyl substituent in the presence of a catalyst as claimed in any one of claims 1 to 8.

10. A process according to claim 9 wherein said mono alkyl-substitute benzene is toluene.

11. A process for the selective production of a para dialkyl-substituted benzene comprising contacting benzene and/or a mono alkyl-substituted benzene having 1-4 carbon atoms in the alkyl substituent with an alkylating agent containing from 1 to 4 carbon atoms in the presence of a catalyst as claimed in any one of claims 1 to 8.

12. A process according to any one claims 9 to 11 and effected at a temperature of 260 to 650°C (500 to 1200°F), a pressure of 1480 to 7000 kPa (200 to 1000 psig), and a weight hourly space velocity of 1/2 to 20.

13. A process according to any one claims 9 to 11 and effected at a temperature of 315 to 480°C (600 to 900°F), a pressure of 2860 to 5620 kPa (400 to 800 psig), and a weight hourly space velocity of 4 to 10.

**Patentansprüche**

1. Katalysator zur selektiven Herstellung von para-dialkylsubstituierten Benzolen, der einen Zeolith umfaßt, der:
   a) einen Zwangsindex von 1 bis 12 aufweist;
   b) aus einer Formungsmischung hergestellt wurde, die ohne organisches Leitmittel ist; und
   c) durch Behandlung mit einer Organosiliciumverbindung modifiziert wurde.

2. Katalysator nach Anspruch 1, worin der Zeolith ZSM-5 umfaßt.

3. Katalysator nach Anspruch 1 oder 2, worin der Zeolith ein Siliciumdioxid/Aluminiumoxid-Molverhältnis von weniger als 55:1 aufweist.

4. Katalysator nach Anspruch 1 oder 2, worin der Zeolith ein Siliciumdioxid/Aluminiumoxid-Molverhältnis von weniger als 30:1 aufweist.

5. Katalysator nach einem der vorstehenden Ansprüche, worin der Zeolith eine Diffusionsgeschwindigkeitskonstante ($D/r^2$) von weniger als 150 $s^{-1}$ x $10^{-6}$ zeigt.

6. Katalysator nach einem der vorstehenden Ansprüche, worin die Modifizierung des Zeolith das Erwärmen des mit Organosilicium modifizierten Zeolith in einer oxidierenden Umgebung umfaßt, um die Organosiliciumverbindung in Siliciumdioxid umzuwandeln.

7. Katalysator nach Anspruch 6 worin der modifizierte Zeolith 0,5 bis 30 Gew.-% Siliciumdioxid enthält.

8. Katalysator nach einem der vorstehenden Ansprüche, der ein Bindemittel umfaßt, das aus Siliciumdioxid, Germaniumoxid, Titandioxid und Zirconiumdioxid ausgewählt ist.

9. Verfahren zur selektiven Herstellung von para-dialkylsubstituiertem Benzol durch Disproportionierung von monoalkylsubstituiertem Benzol mit 1 bis 4 Kohlenstoffatomen im Alkylsubstituenten in Gegenwart eines Katalysators nach einem der Ansprüche 1 bis 8.

10. Verfahren nach Anspruch 9, worin das monoalkylsubstituierte Benzol Toluol ist.

11. Verfahren zur selektiven Herstellung von para-dialkylsubstituiertem Benzol, das den Kontakt von Benzol und/oder monoalkyl-substituiertem Benzol mit 1 bis 4 Kohlenstoffatomen im Alkylsubstituenten mit einem Alkylierungsmittel, das 1 bis 4 Kohlenstoffatome enthält, in Gegenwart eines Katalysators nach einem der Ansprüche 1 bis 8 umfaßt.

12. Verfahren nach einem der Ansprüche 9 bis 11, das bei einer Temperatur von 260 bis 650°C (500 bis 1200°F), einem Druck von 1480 bis 7000 kPa (200 bis 1000 psig) und einer stündlichen Gewichts -Raum-Geschwindigkeit von 1/2 bis 20 durchgeführt wird.

13. Verfahren nach einem der Ansprüche 9 bis 11, das bei einer Temperatur von 315 bis 480°C (600 bis 900°F), einem Druck von 2860 bis 5620 kPa (400 bis 800 psig) und einer stündlichen Gewichts-Raum-Geschwindigkeit von 4 bis 10 durchgeführt wird.

## Revendications

1. Catalyseur pour la production sélective de benzènes p-disubstitués avec des groupes alkyles, comprenant une zéolite qui:
   a) a un indice de contrainte de 1 à 12;
   b) a été produite à partir d'un mélange de formation exempt d'agent directeur organique; et
   c) a été modifiée par traitement avec un composé organosilicique.

2. Catalyseur suivant la revendication 1, dans lequel cette zéolite comprend une ZSM-5.

3. Catalyseur suivant les revendications 1 ou 2, dans lequel la zéolite a un rapport molaire silice/alumine inférieur à 55/1.

4. Catalyseur suivant les revendications 1 ou 2, dans lequel la zéolite a un rapport molaire silice/alumine inférieur à 30/1.

5. Catalyseur suivant l'une quelconque des revendications précédentes, dans lequel la zéolite présente une constante de vitesse de diffusion ($D/r^2$) inférieure à environ 150 $s^{-1}x10^{-6}$.

6. Catalyseur suivant l'une quelconque des revendications précédentes, dans lequel la modification de la zéolite comprend le chauffage de la zéolite modifiée par un composé organosilicique dans un milieu oxydant pour convertir le composé organosilicique en silice.

7. Catalyseur suivant la revendication 6, dans lequel la zéolite modifiée contient 0,5 à 30% en poids de silice.

8. Catalyseur suivant l'une quelconque des revendications précédentes, incluant un liant choisi parmi la silice, le germanium, le titane et le zirconium.

9. Procédé pour la production sélective d'un benzène p-disubstitué avec des groupes alkyles par disproportionation d'un benzène monosubstitué avec un groupe alkyle ayant 1 à 4 atomes de carbone, en présence d'un catalyseur suivant l'une quelconque des revendications 1 à 8.

10. Procédé suivant la revendication 9, dans lequel ce benzène monoalkyl-substitué est le toluène.

11. Procédé pour la production sélective d'un benzène p-disubstitué avec des groupes alkyles, comprenant la mise en contact du benzène et/ou d'un benzène monosubstitué avec un groupe alkyle ayant 1 à 4 atomes de carbone, avec un agent alkylant contenant 1 à 4 atomes de carbone en présence d'un catalyseur suivant l'une quelconque des revendications 1 à 8.

12. Procédé suivant l'une quelconque des revendications 9 à 11, effectué à une température de 260 à 650°C

(500 à 1200°F), une pression de 1480 à 7000 kPa (200 à 1000 psig) et une vitesse spatiale horaire pondérale de 0,5 à 20.

**13.** Procédé suivant l'une quelconque des revendications 9 à 11, effectué à une température de 315 à 480°C (600 à 900°F), une pression de 2860 à 5620 kPa (400 à 800 psig) et une vitesse spatiale horaire pondérale de 4 à 10.

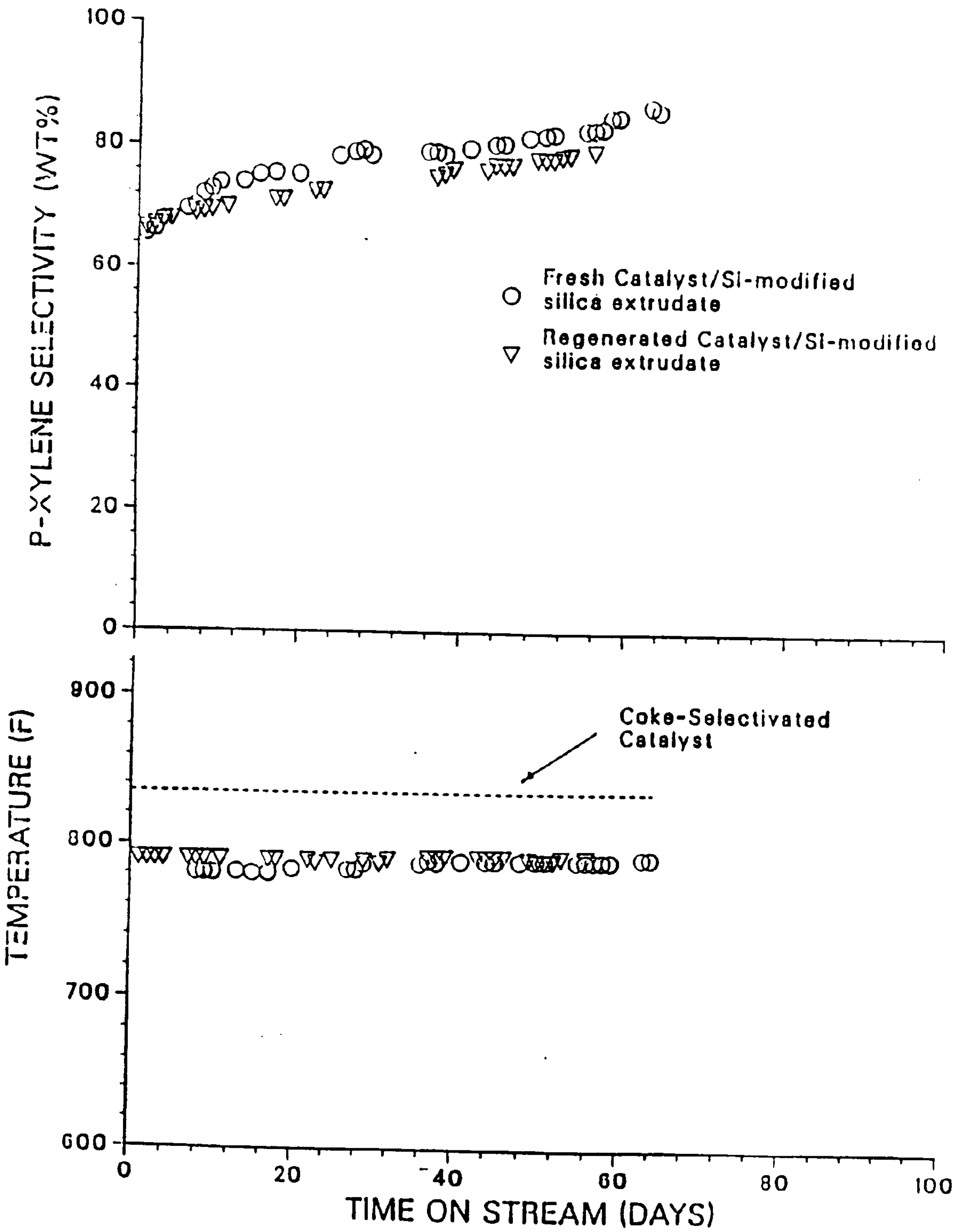
P-XYLENE SELECTIVITY (WT%)
100
80
60
40
20
0
Fresh Catalyst/Si-modified silica extrudate
Regenerated Catalyst/Si-modified silica extrudate
TEMPERATURE (F)
900
800
700
600
Coke-Selectivated Catalyst
0
20
40
60
80
100
TIME ON STREAM (DAYS)